# EUROPÄISCHE PATENTSCHRIFT

(11) **EP 0 902 652 B1**
(45) Veröffentlichungstag und Bekanntmachung des Hinweises auf die Patenterteilung: **28.03.2001**
(21) Anmeldenummer: 95924848.5
(22) Anmeldetag: 11.07.1995
(51) Int. Cl.: A61B 17/34, A61M 25/01, A61B 1/00

(54) **MEDIZINISCHES INSTRUMENT MIT EINEM MANIPULATOR**
MEDICAL INSTRUMENT WITH A MANIPULATOR
INSTRUMENT A USAGE MEDICAL POURVU D'UN MANIPULATEUR

(30) Priorität: 11.07.1994 DE 4424327
(43) Veröffentlichungstag der Anmeldung: 24.03.1999
(73) Patentinhaber: Karl Storz GmbH & Co. KG, 78532 Tuttlingen (DE)
(72) Erfinder: BARKI, Gérard, CH-1206 Genève (CH); STORZ, Karl, D-78532 Tuttlingen (DE)
(74) Vertreter: Münich, Wilhelm, Dr.
(86) Internationale Anmeldenummer: DE9500899
(87) Internationale Veröffentlichungsnummer: WO9601592

(56) Entgegenhaltungen:
- DE-A- 4 010 573
- FR-A- 450 345
- US-A- 4 383 532
- US-A- 4 616 648
- US-A- 5 078 658
- US-A- 5 259 587

## Beschreibung

### Technisches Gebiet

Die Erfindung bezieht sich auf ein medizinisches Instrument und insbesondere auf ein endoskopisches Instrument mit einem Manipulator gemäß dem Oberbegriff des Anspruchs 1. Ein solches Instrument ist aus FR 450 345 A bekannt.

### Stand der Technik

In einer Reihe von Fällen ist es erforderlich, zwei insbesondere medizinische Instrumente gegeneinander bzw. einen Instrumententeil relativ zu einem Instrumenten-Grundkörper präzise zu verschieben. Ein Beispiel hierfür ist die Verschiebung einer Zange in dem Arbeitskanal eines Endoskopschaftes. Weitere Beispiele sind die Verschiebung einer Laserfaser oder eines Führungsdrahtes in einem Katheder oder eines flexiblen dünnen oder ultradünnen Endoskops relativ zu einem Handstück.

Bekannte Manipulatoren weisen einen Führungskanal auf, an dem wenigstens ein Antriebsrad bzw. -rolle und wenigstens ein Führungsrad bzw. -rolle angeordnet sind, deren jeweilige Umfangsfläche mit der Außenkontur eines Instrumententeils derart in Eingriff bringbar ist, daß dieser Instrumententeil in Richtung seiner Längsachse relativ zu dem Manipulator vor und zurück verschiebbar ist. Beispielsweise können Manipulatoren zur Relativverschiebung zweier Instrumente in Richtung deren Längsachse so aufgebaut sein, daß ein (inneres Instrument) teilweise in einen Arbeitskanal des anderen Instruments (äußeres Instrument) eingesetzt ist.

In der Praxis ist es nun häufig erforderlich, in einem Endoskopschaft oder einem Katheder nacheinander Instrumente unterschiedlichen Durchmessers handzuhaben, d.h. zu verschieben. Weiterhin können flexible Endoskope - u.a. aufgrund von Fertigungstoleranzen - zumindest geringfügig schwankende Außendurchmesser aufweisen; dies erschwert die schlupffreie bzw. klemmungsfreie Handhabung mit bekannten Manipulatoren beträchtlich.

Aus der FR-A-450 345 ist ein medizinisches, insbesondere endoskopisches Instrument bekannt, dass einen Manipulator mit einem Führungskanal aufweist, an dem wenigstens ein Antriebs- und wenigstens ein Führungsrad bzw. eine Antriebs- bzw. Führungsrolle angeordnet sind, deren Umfangsfläche mit der Außenkontur eines Instrumententeils derart in Eingriff gebracht werden kann, dass dieser Instrumententeil in Richtung seiner Längsachse relativ zum Manipulator vor und zurück verschiebbar ist, wobei zur Anpassung an unterschiedliche Außendurchmesser der zu verschiebenden Instrumententeile wenigstens eines der Räder in Richtung senkrecht zum Führungskanal bewegbar ist. Eine schnelle und einfache Verstellung des Rades ist bei diesem Instrument nicht möglich.

Die bekannten Manipulatoren, wie sie vorstehend beschrieben worden sind, sind darüberhinaus nur bedingt für den medizinischen Einsatz geeignet, da sie aufgrund ihres komplizierten Aufbaus nicht einfach zu sterilisieren sind.

### Darstellung der Erfindung

Der Erfindung liegt die Aufgabe zugrunde, einen Manipulator zur Verschiebung eines Instrumententeils relativ zu einem Manipulatorgrundkörper bzw. zur Relativverschiebung zweier Instrumente anzugeben, der die Handhabung bzw. die Verschiebung von Instrumente mit unterschiedlichem Durchmesser vereinfacht, und der darüberhinaus leicht sterilisierbar ist.

Eine erfindungsgemäße Lösung dieser Aufgabe ist im Patentanspruch 1 angegeben. Weiterbildungen der Erfindung, sind Gegenstand der Ansprüche 2-12.

Selbstverständlich ist es möglich, daß der Manipulator für den Vorschub und die Führung des zu verschiebenden Instrumententeils mehrere Räder bzw. Rollen, beispielsweise drei Rollen aufweist, da insbesondere durch drei Rollen eine sichere Geradführung" gewährleistet ist. Bei der im Anspruch 2 gekennzeichneten besonders einfachen Ausgestaltung weist der Manipulator zwei sich gegenüberliegende Räder auf.

Zur Vermeidung von Schlupf und/oder zum Ausgleich von fertigungsbedingten Durchmesserschwankungen ist es gemäß Anspruch 3 besonders bevorzugt, wenn wenigstens das oder die Antriebsräder in dem Bereich, mit dem sie mit der Außenkontur des zu verschiebenden Instrumententeils in Eingriff stehen, mit einem gummiartigen Material beschichtet sind, so daß ein besonders präzises Arbeiten möglich ist. Das gummiartige Material kann insbesondere ein Siliconkautschuk sein (Anspruch 5).

Im Anspruch 4 ist eine besonders bevorzugte Ausgestaltung gekennzeichnet, bei der aufgrund der "großflächigen" Anlage der Räder an dem handzuhabenden Instrumententeil ein besonders sicherer und präziser Vorschub des zu verschiebenden Instrumententeils gewährleistet ist:

Bei dieser Ausgestaltung sind die Räder an ihrem Umfang mit zwei O-Ringen versehen sind. Ferner sind die Räder derart angeordnet, daß jeweils zwei O-Ringe sich gegenüberliegen, und daß die O-Ringe einen Spalt einschließen, den der Instrumententeil durchsetzt. Die Verwendung von O-Ringen hat den weiteren Vorteil, daß diese leicht ausgetauscht und/oder gereinigt bzw. sterilisiert werden können; dies ist insbesondere im medizinischen Bereich von Vorteil.

Ferner ist es bevorzugt, wenn der Träger in den zur Schwenkachse senkrechten Schnitten eine kreisförmige Außenkontur hat, und um wenigstens 360° insbesondere von Hand drehbar ist Anspruch 6. Damit kann der Träger "nach links oder rechts" soweit gedreht werden, bis das an ihm exzentrisch angelenkte Rad an dem jeweils vor- und zurückzuschiebenden Instrument bzw. Instrumententeil ausreichend fest anliegt, so daß das Instrument ohne Schlußf verschoben werden kann.

Eine Ausgestaltung ist dadurch angegeben, daß bei der zur Anpassung an unterschiedliche Instrumententeil-Durchmesser durch Verschiebung eines der Räder zwei Schrauben vorgesehen sind, die an der Achse dieses Rades bzw. Rolle angreifen. Bei dieser Ausgestaltung ist es bevorzugt, wenn die Achse des verschiebbaren Rades entgegen der Einschraubrichtung durch eine Feder vorgespannt ist.

Unabhängig von der Anlenkung des bewegbaren Rades ist es gemäß Anspruch 7 von Vorteil, wenn das zur Anpassung an unterschiedliche Instrumentendurchmesser bewegbare Rad das Antriebsrad ist. Hierdurch ist es nämlich möglich, durch entsprechendes Drehen bzw. Schwenken des Träger den Anpreßdruck des Antriebsrades direkt einzustellen, so daß immer ein für den Vorschub des Instrumententeils ausreichendes Moment übertragen werden kann und damit auch schwierige Handhabungsaufgaben gelöst werden können.

Weiterhin ist es bevorzugt, wenn gemäß der im Anspruch 8 angegebenen Weiterbildung das Antriebsrad einen wesentlich kleineren Durchmesser als das oder die Führungsräder hat:

Hierdurch ist es nämlich möglich, das Instrument bzw. den Instrumententeil präzise vor- und zurückzuschieben, da für kleine Vorschübe bereits große Drehwinkel des Antriebsrades erforderlich sind, wobei gleichzeitig durch das große Führungrad eine sichere Führung gewährleistet ist.

Das erfindungsgemäße Instrument kann dabei auf verschiedenste Arten aufgebaut sein:

Beispielsweise können die Räder in einem Handstück angeordnet sein, das einen Kanal für den zu verschiebenden Instrumententeil aufweist. Diese Ausgestaltung eignet sich besonders für die Handhabung bzw. die Verschiebung dünner flexibler Endoskope (Anspruch 9).

Bei einer Weiterbildung weist der Manipulator wenigstens an einer Seite des Führungskanals einen Normflansch zur Verbindung mit einem der Instrumente auf:

Dieser Normflansch kann beispielsweise ein Luer-Lock-Flansch oder ein Endoskopschaft-Flansch sein. Durch diese Weiterbildung kann der Manipulator an vorhandenen Kathedern oder Endoskopschäften angebracht werden, ohne daß diese in irgendeiner weise verändert bzw. angepaßt werden müßten.

Selbstverständlich ist es aber auch möglich, den erfindungsgemäßen Manipulator fest mit einem der Instrumente, d.h. dessen Grundkörper zu verbinden bzw. den Manipulator in ein Instrument, wie beispielsweise einen Endoskopschaft zu integrieren.

Unabhängig von den vorstehend beschriebenen Details der Ausgestaltung des Instruments ist es gerade bei medizinischen Anwendungen von Vorteil, wenn der Radträger lösbar in einem Manipulator-Grundkörper und/oder die Welle des Antriebsrades lösbar in dem Träger angebracht ist. Hierdurch ist eine leichte Reinigung und Sterilisierung des Manipulators sichergestellt, da gerade die nicht leicht zu sterilisierenden Teile des Kanals hierdurch leicht zugänglich werden.

Das bei dem ausgebildeten Instrument vorgesehene Antriebsrad kann motorisch - beispielsweise von einer elektronischen Steuereinheit gesteuert - oder von einer Bedienungsperson von Hand - direkt oder über ein Zwischengetriebe - angetrieben werden.

Mit dem Instrument können die verschiedensten Instrumententeile, wie beispielsweise Zangen, Scheren, Laserfasern, Führungsdrähte von Kathedern oder flexible Endoskope vor- und zurückgeschoben werden. In dem Falle, daß flexible Endoskope mit einem flexiblen Bildleiter und einem flexiblen BeleuchtungslichtLeiter gehandhabt werden sollen, ist es bevorzugt, wenn der Manipulator an seinem distalen Ende einen Führungskanal für die flexible Fasereinheit aufweist, der verglichen mit dem Kanal am proximalen Ende Handstück einen wesentlich kleineren Durchmesser hat.

### Kurze Beschreibung der Zeichnung:

Nachstehend wird die Erfindung anhand von Ausführungsbeispielen exemplarisch ohne Beschränkung unter Bezugnahme auf die Zeichnung näher beschrieben, in der zeigen:
- Fig. 1a bis 1d: vier unterschiedliche Schnitte bzw. Ansichten eines ersten Ausführungsbeispiels der Erfindung, und
- Fig. 2a bis 2d: zwei Varianten eines zweiten Ausführungsbeispiels.

### Darstellung von Ausführungsbeispielen

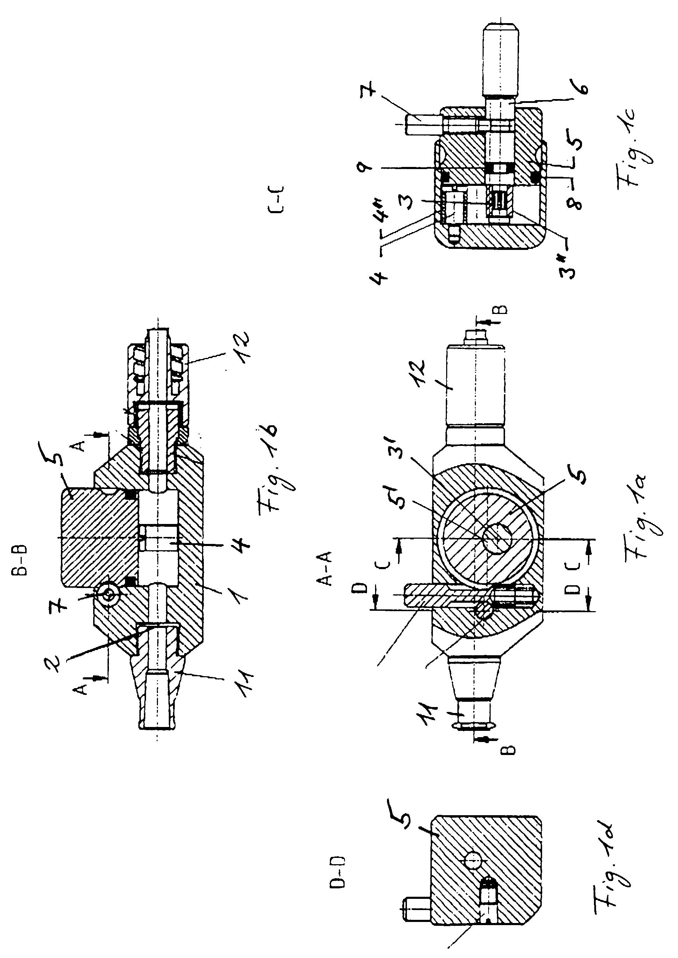

In den Fig. 1a bis 1d sind Schnitte A-A, B-B, C-C und D-D durch den Manipulator eines ersten Ausführungsbeispiels der Erfindung dargestellt.

Der Manipulator weist einen Grundkörper 1 auf, in dem ein Führungskanal 2 vorgesehen ist, in den - in Fig. 1b auf der linken Seite - ein Instrument bzw. ein Instrumententeil eingesetzt und nach rechts vorgeschoben werden kann. Das Instrument, das beispielsweise ein flexibles Endoskop sein kann, ist in den Figuren 1a bis 1d nicht dargestellt.

Zum Vor- und Zurückschieben des Instruments sind an dem Führungskanal 2 ein Antriebsrad 3 und ein Führungsrad 4 angeordnet, deren Umfangsfläche mit der Außenkontur des nicht dargestellten Instrumententeils in Eingriff steht.

Durch die Drehung des Antriebsrades 3 wird das Instrument bzw. der in Richtung seiner Längsachse relativ zu dem Manipulator 1 vor und zurück geschoben.

Zur Anpassung an unterschiedliche Außendurchmesser der zu verschiebenden Instrumententeile ist das Antriebsrad 3 an einer Scheibe 5 angelenkt, deren Drehachse 5' von der Drehachse 3' des Antriebsrades 3 einen Abstand ungleich Null hat. Anders ausgedrückt, ist das Antriebsrad 3 exzentrisch an der Scheibe 5 angebracht. Damit kann der Scheibe 5 "nach links oder rechts" soweit gedreht werden, bis das an ihm exzentrisch angelenkte Antriebsrad 3 an dem jeweils vor- und zurückzuschiebenden und im Kanal 2 befindlichen Instrument bzw. Instrumententeil ausreichend fest anliegt, so daß das Instrument ohne Schlupf präzise verschoben werden kann.

Zum Drehen des Antriebsrades 3 ist dieses - unter Umständen über ein Getriebe oder eine Übersetzung - mit einem Bedienteil 6 verbunden, mittels dem eine Bedienperson das Antriebsrad 3 von Hand drehen kann.

Um die Reinigung bzw. Sterilisierung des aufgebauten Manipulators 1 zu erleichtern, ist die Schreibe 5 für das verschiebbare Antriebsrad 3 lösbar in dem Manipulator-Grundkörper 1 angebracht. Hierzu ist ein in Richtung seiner Längsachse verschiebbarer Zapfen 7 vorgesehen, der eine Profilierung aufweist, die es gestattet, nach einer Verschiebung des Zapfens 7 die Scheibe 5 aus dem Grundkörper 1 des Manipulators herauszunehmen. Zur Abdichtung der Scheibe 5 gegenüber dem Grundkörper 1 ist ein O-Ring 8 vorgesehen. Auch die Welle 6 des Antriebsrades 3 ist mittels eines O-Rings 9 lösbar in die Scheibe 5 eingesteckt.

Weiterhin weist der Manipulator auf beiden Seiten des Führungskanals jeweils einen Normflansch, beispielsweise einen Luer-Lock-Flansch 11 und einen handelsüblichen Endoskopschaft-Flansch 12, wie beispielsweise den Standardflansch der Karl Storz GmbG & Co., Deutschland auf.

Ferner sind das Antriebsrad 3 und das Führungsrad 4 in dem Bereich, mit dem sie mit der Außenkontur des zu verschiebenden Instrumententeils in Eingriff stehen, mit einem gummiartigen Material 3" und 4", wie beispielsweise einem Siliconkautschuck beschichtet, so daß der Schlupf weiter minimiert wird.

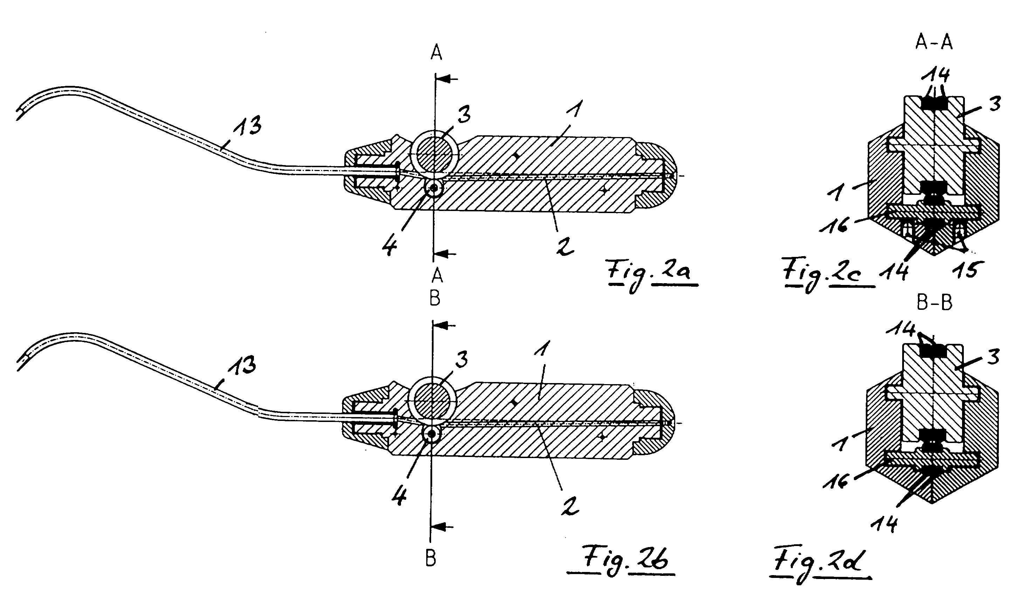

In den Fig. 2a bis 2d sind zwei Varianten eines zweiten Auführungsbeispiels dargestellt, bei dem entsprechende Teile wie in Fig. 1 mit den gleichen Bezeugszeichen versehen sind, so daß auf eine erneute Vorstellung dieser Teile verzichtet wird.

Bei dem zweiten Ausführungsbeispiel ist der Grundkörper 1 als Handstück ausgebildet, in dem der Kanal 2 für den zu verschiebenden Instrumententeil vorgesehen ist. Der Instrumententeil kann dabei insbesondere ein flexibles dünnes Endoskop sein, das in einem Ansatz 13 des Handstücks 1 gefördert wird.

Bei dem zweiten Ausführungsbeispiel sind das Antriebsrad 3 und das Führungsrad 4 an ihrem Umfang mit jeweils zwei 0-Ringen 14 versehen. Die Räder 3 und 4 sind derart angeordnet, daß jeweils zwei 0-Ringe 14 sich gegenüberliegen, und daß die 0-Ringe einen Spalt einschließen, den das Instrument bzw. der Instrumententeil durchsetzt.

Die in den Figuren 2a im Längsschnitt und in Fig. 2c im Querschnitt bei A-A dargestellte Variante unterscheidet sich von der in den entsprechenden Figuren 2b und 2d dargestellten Variante dadurch, daß bei ihr zur Anpassung an unterschiedliche Instrumententeil-Durchmesser zur Verschiebung des Führungsrades 4 zwei Schrauben 15 vorgesehen sind, die an der Achse 16 des Rades 4 angreifen. Die Achse 16 des Führungsrades 4 kann dabei entgegen der Einschraubrichtung der Schrauben 15 durch eine nicht dargestellte Feder vorgespannt sein.

Bei dem in Fig. 2 dargestellten Ausführungsbeispiel wird das Antriebsrad 3 von der Bedienungsperson direktbeispielsweise mit dem Daumen - angetrieben. Aus diesem Grunde hat das Antriebsrad 3 einen größeren Durchmesser als das Führungsrad 4.

## Patentansprüche

1. Medizinisches Instrument und insbesondere endoskopisches Instrument, das einen Manipulator(1) mit einem Führungskanal(2) aufweist, an dem wenigstens ein Antriebs-(3) und wenigstens ein Führungsrad(4) bzw. -rolle angeordnet sind, deren Umfangsfläche mit der Außenkontur eines Instrumententeils derart in Eingriff bringbar ist, daß dieser Instrumententeil in Richtung seiner Längsachse relativ zu dem Manipulator(1) vor und zurück verschiebbar ist, wobei zur Anpassung an unterschiedliche Außendurchmesser der zu verschiebenden Instrumententeile wenigstens eines der Räder(3,4) in Richtung senkrecht zum Führungskanal(2) bewegbar ist,
dadurch **gekennzeichnet,** daß das zur Anpassung an unterschiedliche Instrumententeil-Durchmesser bewegbare Rad(3,4) an einer Scheibe(5) angelenkt ist, die um ihre Mittelachse(5') drehbar ist, und an der exzentrisch die Drehachse des bewegbaren Rades (3') angebracht ist.

2. Instrument nach Anspruch 1,
dadurch **gekennzeichnet,** daß der Manipulator(1) zwei sich gegenüberliegende Räder(3,4) aufweist.

3. Instrument nach Anspruch 1 oder 2,
dadurch **gekennzeichnet,** daß wenigstens eines der Räder(3,4) mit einer elastischen Schicht(3",4") an seiner Außenkontur versehen ist.

4. Instrument nach einem der Ansprüche 1 bis 3,
dadurch **gekennzeichnet,** daß die Räder(3,4) an ihrem Umfang mit zwei aus einem gummiartigen Material bestehenden 0-Ringen(14) versehen sind und daß die Räder(3,4) derart angeordnet sind, daß jeweils zwei O-Ringe(14) sich gegenüberliegen, und daß die O-Ringe(14) einen Spalt einschließen, den der Instrumenteneil durchsetzt.

5. Instrument nach einem der Ansprüche 3 oder 4,
dadurch **gekennzeichnet,** das die elastische Schicht (3",4") bzw. das gummiartige Material der O-Ringe (14) ein Siliconkautschuk ist.

6. Instrument nach einem der Ansprüche 1 bis 5,
dadurch **gekennzeichnet,** daß der Träger(5) in den zur Schwenkachse senkrechten Schnitten eine kreisförmige Außenkontur hat und um wenigstens 360° insbesondere von Hand drehbar ist.

7. Instrument nach einem der Ansprüche 1 bis 6,
dadurch **gekennzeichnet,** daß das zur Anpassung an unterschiedliche Instrumentendurchmesser bewegbare Rad das Antriebsrad(3) ist.

8. Instrument nach einem der Ansprüche 1 bis 7,
dadurch **gekennzeichnet,** daß das Antriebsrad(3) einen wesentlich kleineren Durchmesser als das oder die Führungsräder (4) hat.

9. Instrument nach einem der Ansprüche 1 bis 8,
dadurch **gekennzeichnet,** daß die Räder(3,4) in einem Handstück(1) angeordnet sind, das einen Kanal(2) für den zu verschiebenden Instrumententeil aufweist.

10. Instrument nach einem der Ansprüche 1 bis 9,
dadurch **gekennzeichnet,** daß der Manipulator(1) wenigstens an einer Seite des Führungskanals(2) einen Normflansch(12) zur Verbindung mit einem der Instrumente aufweist.

11. Instrument nach Anspruch 10,
dadurch **gekennzeichnet,** daß der Normflansch ein Luer-Lock-Flansch(11) ist.

12. Instrument nach Anspruch 11,
dadurch **gekennzeichnet,** daß der Normflansch ein handelsüblicher Endkoskopschaft-Flansch und insbesondere ein Bajonett-Flansch ist.

## Claims

1. Medical instrument and endoscopic instrument in particular, comprising a manipulator (1) with a guiding shaft (2) on which at least one driving wheel (3) and at least one guiding wheel or roller (4) is disposed which have each a peripheral surface adapted to be engaged with the outside contour of an instrument part such that this instrument part is displaceable, relative to said manipulator (1) in the forward and backward direction along its longitudinal axis, with at least one of said wheels (3, 4) being movable in a direction orthogonal on said guiding shaft (2) for adaptation to different outside diameters of the instrument parts to be displaced,
**characterised** in that said wheel (3, 4) movable for adaptation to different diameters of the instrument part is articulated on a disk (5) which is rotatable about its centre axis (5') and on which the axle for rotation of said movable wheel (3') is eccentrically mounted.

2. Instrument according to Claim 1,
**characterised** in that said manipulator (1) comprises two opposing wheels (3, 4).

3. Instrument according to Claim 1 or 2,
**characterised** in that at least one of said wheels (3, 4) is provided with a resilient layer (3", 4") on its outside contour.

4. Instrument according to any of the Claims 1 to 3,
**characterised** in that said wheels (3, 4) are provided with two O-rings (14) consisting of a rubber-like material, and that said wheels (3, 4) are disposed in a way that two respective O-rings (14) are opposing each other and that said O-rings (14) enclose a gap through which said instrument part passes.

5. Instrument according to any of the Claims 3 or 4,
**characterised** in that said resilient layer (3", 4") or said rubber-like material, respectively, of said O-rings (14) is a silicone caoutchouc.

6. Instrument according to any of the Claims 1 to 5,
**characterised** in that the carrier (5) presents a circular outside contour in the sections orthogonal on the pivoting axis, and is rotatable through at least 360°, particularly by hand.

7. Instrument according to any of the Claims 1 to 6,
**characterised** in that said wheel movable for adaptation to different diameters of the instruments is said driving wheel (3).

8. Instrument according to any of the Claims 1 to 7,
**characterised** in that said driving wheel (3) has a diameter substantially smaller than that of said guiding wheel or wheels (4).

9. Instrument according to any of the Claims 1 to 8,
**characterised** in that said wheels (3, 4) are disposed in a handpiece (1) including a shaft (2) for the instrument part to be displaced.

10. Instrument according to any of the Claims 1 to 9,
**characterised** in that said manipulator (1) comprises a standard flange (12) at least on one side of said guiding shaft (2) for connection to one of said instruments.

11. Instrument according to Claim 10,
**characterised** in that said standard flange is a Luer lock flange (11).

12. Instrument according to Claim 11,
**characterised** in that said standard flange is a commercially available endoscope shaft flange and a bayonet-type flange in particular.

## Revendications

1. Instrument à médical, et instrument endoscopique en particulier, comprenant un manipulateur (1) à un conduit de guidage (2) sur lequel au moins une roue motrice (3) et au moins une roue ou galet de guidage (4) est disposée, dont chacune a une face périphérique apte à entrer en prise dans le contour extérieur d'une partie d'instrument, de façon que cette partie de l'instrument soit déplaçable relativement audit manipulateur (1) en avant et en arrière selon son axe longitudinal, à au moins une desdites roues (3, 4) étant mobile en sens orthogonal sur ledit conduit de guidage (2) pour l'adaptation aux diamètres extérieurs différents des parties d'instrument à déplacer,
**caractérisé** en ce que ladite roue (3, 4), qui est mobile pour l'adaptation aux diamètres différents de la partie d'instrument, est articulée sur une disque (5), qui est rotative autour de son axe central (5') et sur laquelle l'arbre pour la rotation de ladite roue mobile (3') est monté de façon excentrique.

2. Instrument selon la revendication 1,
**caractérisé** en ce que ledit manipulateur (1) comprend deux roues (3, 4) l'une en face de l'autre.

3. Instrument selon la revendication 1 or 2,
**caractérisé** en ce qu'au moins une desdites roues (3, 4) est munie d'une couche élastique (3", 4") sur son contour extérieur.

4. Instrument selon une quelconque des revendications 1 à 3,
**caractérisé** en ce que lesdites roues (3, 4) sont munies de deux éléments toriques (14) faits en un matériau gommeux, et en ce que lesdites roues (3, 4) sont disposées de façon que deux éléments toriques respectifs (14) se trouvent l'un en face de l'autre et que lesdits éléments toriques (14) renferment un interstice traversé par ladite partie de l'instrument.

5. Instrument selon une quelconque des revendications 3 ou 4,
**caractérisé** en ce que ladite couche élastique (3", 4") ou respectivement ledit matériau gommeux desdits éléments toriques (14) est un caoutchouc au silicone.

6. Instrument selon une quelconque des revendications 1 à 5,
**caractérisé** en ce que le porteur (5) présente un contour extérieur circulaire en sens orthogonales sur son axe de pivotement, en étant apte à être tourné par au moins 360°, en particulier à la main.

7. Instrument selon une quelconque des revendications 1 à 6,
**caractérisé** en ce que ladite roue mobile pour l'adaptation aux diamètres différents des instruments est ladite roue motrice (3).

8. Instrument selon une quelconque des revendications 1 à 7,
**caractérisé** en ce que ladite roue motrice (3) a un diamètre essentiellement plus petit que le diamètre de ladite ou lesdites roues de guidage (4).

9. Instrument selon une quelconque des revendications 1 à 8,
**caractérisé** en ce que lesdites roues (3, 4) sont disposées dans un porte-instrument (1) comprenant un conduit (2) pour la partie d'instrument à déplacer.

10. Instrument selon une quelconque des revendications 1 à 9,
**caractérisé** en ce que ledit manipulateur (1) comprend une flasque normalisée (12) au moins d'un côté dudit conduit de guidage (2) pour sa connexion à un desdits instruments.

11. Instrument selon la revendication 10,
**caractérisé** en ce que ladite flasque normalisée est une flasque du type raccord Luer (11).

12. Instrument selon la revendication 11,
**caractérisé** en ce que ladite flasque normalisée est une flasque de manche d'endoscope commerciale et courante et en particulier une flasque du type à baïonnette.
